(19) Europäisches Patentamt / European Patent Office / Office européen des brevets

(11) **EP 4 491 220 A1**

(12) **EUROPEAN PATENT APPLICATION**
published in accordance with Art. 153(4) EPC

(43) Date of publication:
**15.01.2025 Bulletin 2025/03**

(21) Application number: **23876556.4**

(22) Date of filing: **28.09.2023**

(51) International Patent Classification (IPC):
**A61N 5/10** (2006.01)   **G21K 1/00** (2006.01)

(52) Cooperative Patent Classification (CPC):
**A61N 5/10; G21K 1/00; G21K 1/06**

(86) International application number:
**PCT/CN2023/122407**

(87) International publication number:
**WO 2024/078353 (18.04.2024 Gazette 2024/16)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **10.10.2022   CN 202211232520
05.09.2023   CN 202311136590**

(71) Applicant: **Neuboron Therapy System Ltd.
Xiamen, Fujian 361026 (CN)**

(72) Inventors:
• **LIU, Yuanhao**
  **Nanjing, Jiangsu 211112 (CN)**
• **LU, Weihua**
  **Nanjing, Jiangsu 211112 (CN)**
• **SHU, Diyun**
  **Nanjing, Jiangsu 211112 (CN)**

(74) Representative: **Gunzelmann, Rainer
Wuesthoff & Wuesthoff
Patentanwälte und Rechtsanwalt PartG mbB
Schweigerstraße 2
81541 München (DE)**

(54) **BEAM SHAPING BODY AND NEUTRON CAPTURE THERAPY SYSTEM**

(57)    Provided is a beam shaping body (131) for a neutron capture therapy system (100), comprising: a retarder (1312) used for decelerating neutrons of a neutron beam (N) to epithermal neutrons; a reflector (1313) surrounding the retarder (1312) and used for reflecting neutrons deviating from the neutron beam (N) back to the neutron beam (N) so as to improve the intensity of the neutron beam (N); and an epithermal neutron flux enhancer (1316) used for improving an epithermal neutron flux in the neutron beam (N) and provided in the retarder (1312) and/or in the reflector (1313) and/or between the retarder (1312) and the reflector (1313). Further provided is the neutron capture therapy system (100) comprising the beam shaping body (131), the epithermal neutron flux enhancer (1316) is provided in the beam shaping body (131) to increase the epithermal neutron flux in the neutron beam (N), so that the flux and quality of a neutron source are improved.

**FIG. 12**

**Description**

## TECHNICAL FIELD

The present disclosure relates to the field of radioactive ray irradiation, and in particular to a beam shaping assembly (BSA) and a neutron capture therapy system.

## BACKGROUND

With the development of atomics, the radiotherapy such as the cobalt-60, the linear accelerator, and the electron beam has become one of major means to treat cancers. However, the conventional photon or electron therapy is restricted by physical conditions of radioactive rays. Specifically, while tumor cells are killed, a large number of normal tissues on a beam path are damaged. Due to different sensitivities of the tumor cells for the radioactive rays, the conventional radiotherapy is often undesirable to treat radioresistant malignant tumors (such as glioblastoma multiforme and melanoma).

In order to reduce radiation damages to the normal tissues surrounding the tumor, the target therapy in chemotherapy has been employed in the radiotherapy. For the highly radioresistant tumor cells, the radiotherapy with high relative biological effectiveness (RBE), including the proton therapy, the heavy particle therapy, and the neutron capture therapy, has also been developed actively. With specific aggregation of boron-containing drugs in tumor cells, and in cooperation with accurate neutron beam control, boron neutron capture therapy (BNCT) in neutron capture therapy serves as a better alternative to treat the cancers.

With the specific aggregation of the boron-containing drugs in the tumor cells, and in cooperation with accurate neutron beam irradiation, BNCT is more desirable to treat the cancers than the conventional radiotherapy. In the BNCT, the boron (B-10)-containing drug is injected into the patient. With the strong affinity to the tumor cells, the drug is selectively aggregated to the tumor cells. Then, the neutron beam is irradiated onto a tumor site of the patient. Neutrons cause fissions when captured by $^{10}B$ in the tumor cells, thus generating the alpha particle and the $^{7}Li$ particle, and releasing the lethal radioactive rays. Since the radioactive rays have the short range that is only equivalent to the length of the single tumor cell, the tumor cells can be killed accurately without damaging the surrounding normal cells as much as possible.

The BNCT is also called binary cell therapy, for its efficiency depending on a concentration of the boron-containing drug and a number of thermal neutrons in the tumor cells. Therefore, besides the development of the boron-containing drug, improvement of the flux and quality of a neutron source plays an important role in research of the BNCT.

## SUMMARY

In order to improve the flux and quality of the neutron source, an aspect of the present disclosure provides a BSA for neutron capture therapy. The BSA includes: a moderator configured to slow a neutron in a neutron beam down to an epithermal neutron, where the neutron beam defines a beam axis, and the moderator extends for a first preset length along the beam axis and extends for a first preset width along a radial direction of the beam axis; a reflector surrounding the moderator, and configured to reflect a neutron deviated from the neutron beam back to the neutron beam to improve an intensity of the neutron beam, where at least a portion of the reflector extends for a second preset length along the beam axis and extends outside the moderator for a second preset width along the radial direction of the beam axis; and an epithermal neutron flux enhancer configured to increase an epithermal neutron flux in the neutron beam, and provided in the moderator and/or in the reflector and/or between the moderator and the reflector, where the epithermal neutron flux enhancer extends for a third preset length along the beam axis and extends for a third preset width along the radial direction of the beam axis; and the third preset width is less than a sum of the first preset width and the second preset width. The epithermal neutron flux enhancer in the BSA increases the epithermal neutron flux in the neutron beam, thereby improving the flux and quality of the neutron source.

In an embodiment, the BSA further includes: a beam inlet configured to enter a charged particle beam; and a beam outlet configured to exit the neutron beam; and the beam inlet, the moderator, and the beam outlet are arranged along an extension direction of the beam axis.

In an embodiment, the BSA further includes a radiation shield surrounding the reflector; the radiation shield is configured to shield a leaked neutron and a leaked photon to reduce a dose to a normal tissue in a non-irradiated region; and the radiation shield extends for a fourth preset length along the beam axis and extends outside the reflector for a fourth preset width along the radial direction of the beam axis.

In an embodiment, the reflector is made of Pb, and the moderator is made of at least one of $D_2O$, $AlF_3$, $CaF_2$, $Li_2CO_3$, $MgF_2$, $Al_2O_3$, and a material mixed by Al, $AlF_3$ and LiF at a preset ratio.

In an embodiment, the epithermal neutron flux enhancer is made of Ni.

In an embodiment, the epithermal neutron flux enhancer is a cylindrical structure; the cylindrical structure includes a first side and a second side that are perpendicular to the beam axis, as well as a first wall and a second wall that are enclosed circumferentially around the beam axis; the first side and the second side are respectively provided at two ends of the cylindrical structure in sequence along a direction of the neutron beam; the first side is provided with a first central hole; the first central hole is configured to cooperate with the beam inlet; the second side is provided with a second central hole; and the second central hole is configured to cooperate with the beam outlet.

In an embodiment, the first side and/or the second side of the epithermal neutron flux enhancer of the cylindrical structure is a conical structure narrowed toward the beam axis; a radial size of an outer contour of the first side increases gradually along the direction of the neutron beam; a size of the first central hole is capable of accommodating at least the beam inlet; a radial size of an outer contour of the second side decreases gradually along the direction of the neutron beam; and a size of the second central hole of the second side is capable of accommodating at least the beam outlet.

In an embodiment, the epithermal neutron flux enhancer has a thickness of 1-8 cm; and more preferably, the epithermal neutron flux enhancer has a thickness of 3 -5 cm.

In an embodiment, the epithermal neutron flux enhancer is a conical structure; the conical structure includes a first end and a second end that are perpendicular to the beam axis, as well as a third wall and a fourth wall that are enclosed circumferentially around the beam axis; the first end and the second end are arranged in sequence along a direction of the neutron beam; and the first end and the second end each are an opening.

In an embodiment, a radial size of an outer contour of the epithermal neutron flux enhancer of the conical structure increases gradually along the direction of the neutron beam; the first end is adjacent to an end of the moderator located at an upstream side of the neutron beam; and a size of the first end is capable of accommodating at least the beam inlet.

In an embodiment, a radial size of an outer contour of the epithermal neutron flux enhancer of the conical structure decreases gradually along the direction of the neutron beam; the second end is adjacent to an end of the BSA located at a downstream side of the neutron beam; and a size of the second end is capable of accommodating at least the beam outlet. At the beam outlet, the epithermal neutron flux enhancer is provided as the conical structure, such that more epithermal neutrons are reflected back to the neutron beam to enhance the epithermal neutron flux.

In an embodiment, a central line of the epithermal neutron flux enhancer of the cylindrical structure or the conical structure coincides with the beam axis.

Another aspect of the present disclosure provides a neutron capture therapy system, including: a charged particle beam generation portion configured to generate a charged particle beam; a neutron beam generation portion configured to generate a neutron beam, and including a target and a BSA; and a beam transmission portion configured to transmit the charged particle beam to the neutron beam generation portion, where the charged particle beam is interacted with the target to generate the neutron beam; and the neutron beam is moderated by the BSA to form a desired epithermal neutron beam for neutron capture therapy.

In an embodiment, the BSA includes:

a moderator configured to slow a neutron in the neutron beam down to an epithermal neutron, where the neutron beam defines a beam axis, and the moderator extends for a first preset length along the beam axis and extends for a first preset width along a radial direction of the beam axis;
a reflector surrounding the moderator, and configured to reflect a neutron deviated from the neutron beam back to the neutron beam to improve an intensity of the neutron beam, where at least a portion of the reflector extends for a second preset length along the beam axis and extends outside the moderator for a second preset width along the radial direction of the beam axis; and
an epithermal neutron flux enhancer configured to increase an epithermal neutron flux in the neutron beam, and provided in the moderator and/or in the reflector and/or between the moderator and the reflector, where the epithermal neutron flux enhancer extends for a third preset length along the beam axis and extends for a third preset width along the radial direction of the beam axis; and the third preset width is less than a sum of the first preset width and the second preset width.

## BRIEF DESCRIPTION OF THE DRAWINGS

FIG. 1 is a schematic view of a boron neutron capture reaction;
FIG. 2 illustrates an equation of a nuclear reaction in $^{10}B(n,\alpha)^{7}Li$ neutron capture;
FIG. 3 is a schematic structural view of a neutron capture therapy system according to an embodiment of the present disclosure;
FIG. 4 is a schematic structural view of a BSA according to an embodiment of the present disclosure, where an epithermal neutron flux enhancer is a cylindrical structure, and at least a portion of the epithermal neutron flux enhancer is provided in a moderator;

FIG. 5 is a schematic view of a size of a BSA according to an embodiment of the present disclosure, where an epithermal neutron flux enhancer is a cylindrical structure, and at least a portion of the epithermal neutron flux enhancer is provided in a moderator;

FIG. 6 is a schematic view of a radial size of a BSA according to an embodiment of the present disclosure, where an epithermal neutron flux enhancer is a cylindrical structure, and at least a portion of the epithermal neutron flux enhancer is provided in a moderator;

FIG. 7 is a schematic structural view of an epithermal neutron flux enhancer of a cylindrical structure according to an embodiment of the present disclosure;

FIG. 8 is a sectional view of an epithermal neutron flux enhancer of a cylindrical structure according to an embodiment of the present disclosure;

FIG. 9 is a schematic structural view of a BSA according to an embodiment of the present disclosure, where an epithermal neutron flux enhancer is a cylindrical structure, and provided in a reflector;

FIG. 10 is a schematic view of a radial size of a BSA according to an embodiment of the present disclosure, where an epithermal neutron flux enhancer is a cylindrical structure, and provided in a reflector;

FIG. 11 is a schematic structural view of a BSA according to an embodiment of the present disclosure, where one side of an epithermal neutron flux enhancer of a cylindrical structure is a conical structure;

FIG. 12 is a schematic structural view of a BSA according to an embodiment of the present disclosure, where two sides of an epithermal neutron flux enhancer of a cylindrical structure each are a conical structure;

FIG. 13 is a schematic structural view of an epithermal neutron flux enhancer of a cylindrical structure with two sides each being a conical structure according to an embodiment of the present disclosure;

FIG. 14 is a sectional view of an epithermal neutron flux enhancer of a cylindrical structure with two sides each being a conical structure according to an embodiment of the present disclosure;

FIG. 15 (a) illustrates a change trend of $\Phi_{ep}$ over a thickness of an epithermal neutron flux enhancer according to an embodiment of the present disclosure;

FIG. 15 (b) illustrates a change trend of $J_{ep}/\Phi_{ep}$ over a thickness of an epithermal neutron flux enhancer according to an embodiment of the present disclosure;

FIG. 15 (c) illustrates a change trend of $D_f/\Phi_{ep}$ over a thickness of an epithermal neutron flux enhancer according to an embodiment of the present disclosure;

FIG. 15 (d) illustrates a change trend of $D_\gamma/\Phi_{ep}$ over a thickness of an epithermal neutron flux enhancer according to an embodiment of the present disclosure;

FIG. 15 (e) illustrates a change trend of $\Phi_{th}/\Phi_{ep}$ over a thickness of an epithermal neutron flux enhancer according to an embodiment of the present disclosure;

FIG. 16 is a schematic structural view of a B SA according to an embodiment of the present disclosure, where an epithermal neutron flux enhancer is a conical structure, and is located at an upstream side of the BSA along a direction of a neutron beam;

FIG. 17 is a schematic view of a size of a BSA according to an embodiment of the present disclosure, where an epithermal neutron flux enhancer is a conical structure, and is located at an upstream side of the BSA along a direction of a neutron beam;

FIG. 18 is a schematic view of a radial size of a BSA according to an embodiment of the present disclosure, where an epithermal neutron flux enhancer is a conical structure, and is located at an upstream side of the BSA along a direction of a neutron beam;

FIG. 19 is a schematic structural view of an epithermal neutron flux enhancer of a conical structure according to an embodiment of the present disclosure;

FIG. 20 is a sectional view of an epithermal neutron flux enhancer of a conical structure according to an embodiment of the present disclosure;

FIG. 21 is a schematic structural view of a BSA according to an embodiment of the present disclosure, where an epithermal neutron flux enhancer is a conical structure, and is located at a downstream side of the BSA along a direction of a neutron beam; and

FIG. 22 is a schematic structural view of a BSA according to an embodiment of the present disclosure, where an epithermal neutron flux enhancer is a conical structure, and is located at an upstream side and a downstream side of the BSA along a direction of a neutron beam.

In the figures:

100: BNCT system, 11: charged particle generation portion, 111: ion source, 112: accelerator, 12: beam transmission portion, 13: neutron beam generation portion, 131: BSA, 1311: beam inlet, 1312: moderator, 1313: reflector, 1314: beam outlet, 1315: radiation shield, 1316: epithermal neutron flux enhancer, 1316a: first side, 1316b: second side, 1316c: first wall, 1316d: second wall, 1316e: first end, 1316f: second end, 1316g: third wall, 1316h: fourth wall, 132: collimator, 133: target, 200: irradiated body, 20: treatment table, M: affected site, L1: first preset length, L2: second preset length, L3: third preset length, L4: fourth preset length, D1: first preset width, D2: second preset width, D3: third preset width, D4: fourth preset width, and X: beam axis.

## DETAILED DESCRIPTION OF THE EMBODIMENTS

To make the foregoing objectives, features, and advantages of the present disclosure clearer and more comprehensible, the specific implementations of the present disclosure are described in detail below with reference to the drawings. The following describes many details in order to provide a thorough understanding of the present disclosure. However, the present disclosure can be implemented in many other ways other than those described herein, and those skilled in the art can make similar improvements without departing from the connotation of the present disclosure, and thus the present disclosure is not limited to the specific embodiments disclosed below.

In the description of the present disclosure, the terms "central", "longitudinal", "transverse", "length', "width", "thickness", "upper", "lower", "front", "back", "left", "right", "vertical", "horizontal", "top", "bottom", "inner", "outer", "clockwise", "anticlockwise", "axial", "radial" and "circumferential" etc. are used to indicate orientations shown in the accompanying drawings. It should be noted that these terms are merely intended to facilitate a simple description of the present disclosure, rather than to indicate or imply that the mentioned apparatus or elements must have the specific orientation or be constructed and operated in the specific orientation. Therefore, these terms may not be construed as a limitation to the present disclosure.

In addition, the terms "first" and "second" are merely intended for a purpose of description, and shall not be understood as an indication or implication of relative importance or implicit indication of a quantity of indicated technical features. Therefore, a feature limited by "first" or "second" may explicitly or implicitly include at least one such feature. In the descriptions about the present disclosure, "a plurality of' means at least two, for example, two or three, unless otherwise specifically limited.

In the present disclosure, unless otherwise clearly limited, the terms "installation", "interconnection", "connection" and "fixation" etc. are intended to be understood in a broad sense. For example, the "connection" may be a fixed connection, a removable connection or an integral connection; may be a mechanical connection or an electrical connection; may be a direct connection or an indirect connection using a medium; and may be a communication or an interaction between two elements, unless otherwise clearly specified and limited. Those of ordinary skill in the art may understand specific meanings of the above terms in the present disclosure based on a specific situation.

In the present disclosure, unless otherwise clearly specified and limited, when it is described that a first feature is "above" or "below" a second feature, it indicates that the first and second features are in direct contact or the first and second features are in indirect contact through a medium. In addition, when it is described that the first feature is "over", "above" and "on" the second feature, it indicates that the first feature is directly or obliquely above the second feature, or simply indicates that an altitude of the first feature is higher than that of the second feature. When it is described that the first feature is "under", "below" or "beneath" the second feature, it indicates that the first feature is directly or obliquely under the second feature or simply indicates that an altitude of the first feature is lower than that of the second feature.

It should be noted that when a component is "fixed" or "provided" on another component, the component may be fixed on the another component directly or via an intermediate component. When a component is connected to another component, the component may be connected to the another component directly or via an intermediate component. The terms "vertical", "horizontal", "upper", "lower", "left", "right" and other similar expressions used herein are for illustrative purposes only, rather than to mean the only implementation.

Neutron capture therapy, particularly BNCT, has been applied increasingly in recent years as an effective method to treat the cancers. Neutrons in the BNCT can be provided by a nuclear reactor or an accelerator. In an embodiment of the present disclosure, the accelerator is used as an example to provide the neutrons for the BNCT. Basic components in the BNCT using the accelerator usually include the accelerator configured to accelerate charged particles (such as photons and deuterons), a target, a heat removal system, and a BSA. The accelerated charged particles are interacted with a metal target to generate the neutrons. An appropriate nuclear reaction is selected according to characteristics such as a desired neutron yield and energy, available energies of the accelerated charged particles, a current, physical and chemical properties of the metal target, or the like. Nuclear reactions commonly discussed include $^7$Li(p,n)$^7$Be and $^9$Be(p,n)$^9$B, both of which are endothermic reactions. Energy thresholds of the two nuclear reactions are respectively 1.881 MeV and 2.055 MeV. Epithermal neutrons with an energy level of keV are considered as an ideal neutron source in the BNCT. Theoretically, if the photons with energies slightly higher than the threshold are used to bombard the lithium target, neutrons with relative low energies can be generated, and can be used clinically without excessive moderation. However, the target made of the lithium (Li) and beryllium (Be) has a small action cross section with the photons at the threshold energy. In order to generate an enough large neutron flux, photons with high energies are usually selected to trigger the nuclear reaction.

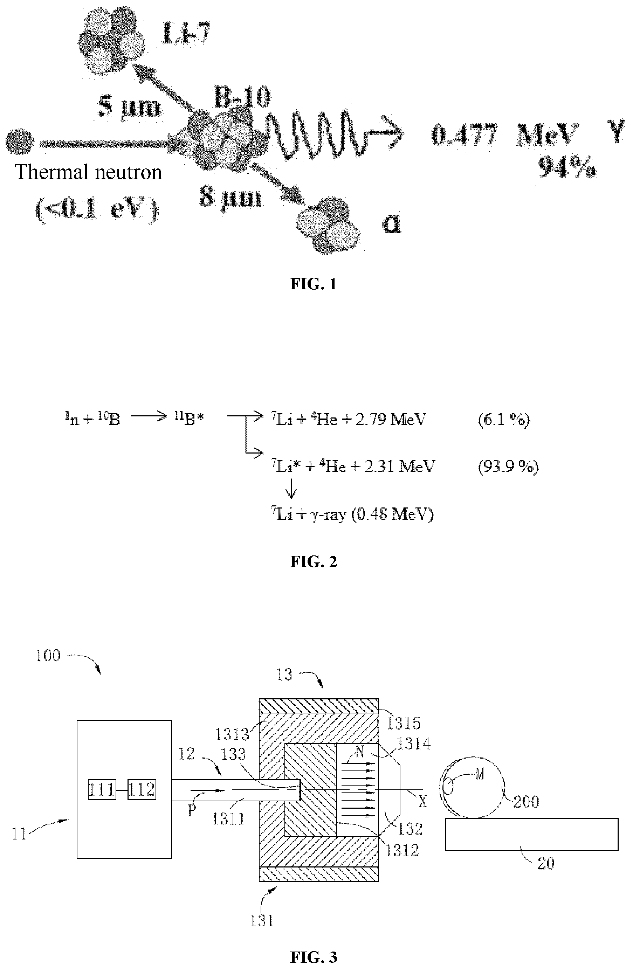

According to the BNCT, with a large capture cross section of the boron ($^{10}$B)-containing drug for thermal neutrons, and through the $^{10}$B(n,$\alpha$)$^7$Li neutron capture reaction and the nuclear fission reaction, $^4$He and $^7$Li heavy charged particles

are generated. FIG. 1 and FIG. 2 respectively illustrate a schematic view of a boron neutron capture reaction and an equation of a nuclear reaction in $^{10}B(n,\alpha)^{7}Li$ neutron capture. The two heavy charged particles have an average energy of about 2.33 MeV, and have characteristics of the high linear energy transfer (LET), and the short range. The alpha particle has the LET of 150 keV/$\mu$m, and the range of 8 $\mu$m. The $^{7}Li$ heavy charged particle has the LET of 175 keV/$\mu$m, and the range of 5 $\mu$m. The total range of the two particles is approximately equivalent to a cell size, such that the radiation damage to an organism can be limited to a cell level. When the boron-containing drug is aggregated to the tumor cells selectively, and an appropriate neutron source is provided, a purpose of locally killing the tumor cells on premise of no serious damage to normal tissues can be achieved.

No matter whether a neutron source in the BNCT comes from the nuclear reactor or the accelerator, a mixed radiation field is generated by the nuclear reaction between the charged particles and the target, that is, the generated beam includes neutrons and photons having energies from low to high. As for the BNCT on deep-seated tumors, except the epithermal neutrons, the more the remaining radioactive rays, the greater the proportion causing non-selective dose deposition in the normal tissues. Therefore, the radioactive rays causing the unnecessary dose deposition should be reduced as much as possible.

For neutron sources in the clinical BNCT, the International Atomic Energy Agency (IAEA) provides five suggestions for beam quality factors in air. The five suggestions can be used to compare advantages and disadvantages of different neutron sources, and used as a reference basis to select a neutron generation method and design the BSA. The five suggestions are as follows:

$$\text{Epithermal neutron flux} > 1*10^{9}\text{n/cm}^{2}\text{s}$$

$$\text{Fast neutron contamination} < 2*10^{-13}\text{Gy-cm}^{2}/\text{n}$$

$$\text{Photon contamination} < 2*10^{-13}\text{Gy-cm}^{2}/\text{n}$$

$$\text{Thermal to epithermal neutron flux ratio} < 0.05$$

$$\text{Epithermal neutron current to flux ratio} > 0.7$$

Note: The epithermal neutrons have an energy range between 0.5 eV to 10 keV, the thermal neutrons have an energy range of less than 0.5 eV, and the fast neutrons have an energy range of greater than 10 keV.

Different classification standards may be provided for the energy range of the epithermal neutrons in different fields. For example, the epithermal neutrons have an energy range between 0.5 eV to 40 keV, the thermal neutrons have an energy range of less than 0.5 eV, and the fast neutrons have an energy range of greater than 40 keV.

1. Epithermal neutron flux $\Phi_{ep}$:

The neutron flux and the concentration of the boron-containing drug in the tumor determine the clinical treatment time. If the concentration of the boron-containing drug in the tumor is high enough, lower requirements may be imposed on the neutron flux. Conversely, if the concentration of the boron-containing drug in the tumor is low, high-flux epithermal neutrons are required to deliver an enough dose to the tumor. According to the IAEA, the epithermal neutron flux is greater than $1*10^{9}\text{n/cm}^{2}\text{s}$. For the existing boron-containing drug, the treatment time of the neutron beam may be controlled within one hour approximately. With the short treatment time, besides the advantages in patient positioning and comfort level, limited detention time of the boron-containing drug in the tumor may also be effectively used.

2. Fast neutron contamination $D_{f}/\Phi_{ep}$:

The unnecessary dose of the fast neutrons to the normal tissues is considered as contamination. This dose is positively correlated with the neutron energy. Thus, the fast neutrons in the neutron beam should be reduced as much as possible. The fast neutron contamination is defined as a fast-neutron dose in the unit epithermal neutron flux. According to the IAEA, the fast neutron contamination should be less than $2*10^{-13}\text{Gy-cm}^{2}/\text{n}$.

3. Photon contamination (gamma-ray contamination) $D_\gamma/\Phi_{ep}$:

With strongly penetrating radiation, gamma-rays will cause dose deposition non-selectively to all tissues on the beam path. To design the neutron beam, it is essential to reduce the gamma-rays. The gamma-ray contamination is defined as a gamma-ray dose in the unit epithermal neutron flux. According to the IAEA, the gamma-ray contamination should be less than $2*10^{-13}$Gy-cm$^2$/n.

4. Thermal to epithermal neutron flux ratio $\Phi_{th}/\Phi_{ep}$:

Due to fast decay and poor penetrability of the thermal neutrons, energies of the thermal neutrons in the body are largely deposited on skin tissues. Except that epidermal tumors such as melanoma are required to use the thermal neutrons as a neutron source in the BNCT, the thermal neutrons should be reduced for deep-seated tumors such as brain tumor. According to the IAEA, the thermal to epithermal neutron flux ratio should be less than 0.05.

5. Epithermal neutron current to flux ratio $J_{ep}/\Phi_{ep}$:

The epithermal neutron current to flux ratio indicates directivity of the beam. The greater the epithermal neutron current to flux ratio, the better the forward directivity of the neutron beam. The neutron beam with the forward directivity can reduce a dose to the surrounding normal tissues due to neutron diffusion, and further improve the treatable depth and the positioning flexibility. According to the IAEA, the epithermal neutron current to flux ratio should be greater than 0.7.

Monte Carlo N-particle (MCNP) software (a general-purposed software package developed by the Los Alamos National Laboratory based on the Monte Carlo method and used for calculating a transport problem of the neutron, photon, charged particle or coupled neutron/photon/charged particle in the three-dimensional (3D) complex geometric structure) is used to calculate quality of the neutron beam in air in the embodiment of the present disclosure.

Referring to FIG. 3, FIG. 3 is a schematic structural view of a neutron capture therapy system according to an embodiment of the present disclosure. The neutron capture therapy system in the embodiment is preferably a BNCT system 100. The neutron beam N generated by a neutron generation portion is irradiated onto an affected site M of an irradiated body 200 on a treatment table 20. The neutron beam N and the boron (B-10)-containing drug at the affected site M take place the above-mentioned boron neutron capture reaction, thereby killing tumor cells or other substances at the affected site M to obtain the treatment effect.

The BNCT system 100 includes a charged particle beam generation portion 11, a beam transmission portion 12, and a neutron beam generation portion 13. The charged particle beam generation portion 11 is configured to generate a charged particle beam P such as a proton beam. The beam transmission portion 12 is configured to transmit the charged particle beam P to the neutron beam generation portion 13. The neutron beam generation portion 13 is configured to generate the neutron beam N for treatment and irradiate the neutron beam onto the irradiated body 200 on the treatment table 20. The charged particle beam generation portion 11 includes an ion source 111 and an accelerator 112. The ion source 111 is configured to generate charged particles, such as H$^-$, protons, and deuterons. The accelerator 112 is configured to accelerate the charged particles generated by the ion source 111 to obtain the charged particle beam P with a desired energy, such as the proton beam. The accelerator 112 may be a linear accelerator, a cyclotron, a synchrotron or a synchrocyclotron. The neutron beam generation portion 13 includes a BSA 131, a collimator 132, and a target 133. The charged particle beam P generated by the accelerator 112 is transmitted to the neutron beam generation portion 13 through the beam transmission portion 12, and interacted with the target 133 to generate neutrons. Through adjustment of the BSA 131 and the collimator 132 on the beam quality, the neutron beam N for the treatment is formed and irradiated onto the irradiated body 200 on the treatment table 20.

The BSA 131 can be configured to adjust quality of the neutron beam N. The collimator 132 is configured to gather the neutron beam N, such that the neutron beam N has a high targeting ability in the treatment. The BSA 131 further includes a beam inlet 1311, a moderator 1312, a reflector 1313 surrounding the moderator 1312, and a beam outlet 1314.

According to the BNCT system 100, the charged particle beam P generated by the ion source 111 is accelerated by the accelerator 112, and the charged particle beam P enters the BSA 131 through the beam inlet 1311. The beam inlet 1311 includes one portion accommodated in the moderator 1312, and the other portion accommodated in the reflector 1313.

The ideal target 133 features a high neutron yield, a capability of generating neutrons with energies close to the energy range of epithermal neutrons, no excessive long-range radiation, safety, a cheap cost, an easy operation, a high temperature resistance, etc. However, as a matter of fact, a nuclear reaction meeting all requirements cannot be found. The target 133 in the embodiment is made of a material meeting the above requirements as much as possible, such as Li or Be. As is known to those skilled in the art, the target 133 may also be made of a metal material other than the Li and the Be, such as Ta or W or an alloy thereof. As a preferred embodiment, the target 133 is made of the Li, and accommodated in the moderator 1312. The charged particle beam P is accelerated sufficiently to overcome a Coulomb repulsive force of the nucleus of the target 133, and takes place the $^7Li(p,n)^7Be$ nuclear reaction with the target 133 to generate neutrons. The neutrons form the neutron beam N. The neutron beam N defines a beam axis X. The energy spectrum of the neutrons generated by the charged particle beam P and the target 133 is very wide. Except that epithermal neutrons meet the treatment requirement, neutrons and photons of other types are to be reduced as much as possible, so as not to hurt the operator or the irradiated body. Hence, concerning neutrons from the target 133, energies (>10 keV) of fast neutrons are adjusted by the moderator 1312 to an energy range (0.5 eV to 10 keV) of the epithermal neutrons, and the thermal neutrons (<0.5 eV) are reduced as much as possible.

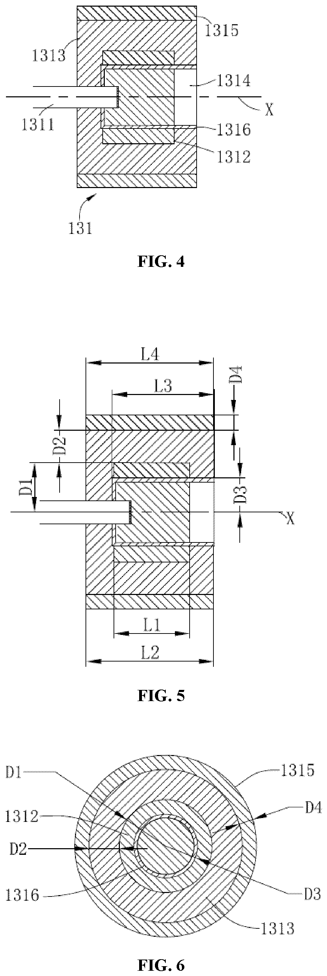

Referring to FIG. 5, the moderator 1312 extends for a first preset length L1 along the beam axis X and extends for a first preset width D1 along a radial direction of the beam axis X. The moderator 1312 is made of a material having a large action cross section with the fast neutrons but a small action cross section with the epithermal neutrons. In the embodiment, the moderator 1312 is made of at least one of $D_2O$, $AlF_3$, $CaF_2$, $Li_2CO_3$, $MgF_2$, $Al_2O_3$, and a material mixed by Al, $AlF_3$ and LiF at a preset ratio. The material mixed by the Al, the $AlF_3$ and the LiF at the preset ratio is preferably a Fluental material.

The reflector 1313 surrounds the moderator 1312, and is configured to reflect a neutron diffused through the moderator 1312 back to the neutron beam N to improve an intensity of the neutron beam N. At least a portion of the reflector 1313 extends for a second preset length L2 along the beam axis X and extends outside the moderator 1312 for a second preset width D2 along the radial direction of the beam axis X. The reflector 1313 is made of a material with a strong neutron reflectivity. In the embodiment, the reflector 1313 is made of Pb.

In the embodiment, the BSA 131 further includes the beam outlet 1314 and a radiation shield 1315 surrounding the reflector 1313. The radiation shield 1315 is configured to shield a leaked neutron and a leaked photon to reduce a dose to a normal tissue in a non-irradiated region. The radiation shield 1315 extends for a fourth preset length L4 along the beam axis X and extends outside the reflector 1313 for a fourth preset width D4 along the radial direction of the beam axis X. The radiation shield 1315 is flush with the reflector 1313 at the beam outlet 1314. It is to be noted that the radiation shield is described with the fourth preset length L4 and the fourth preset width D4 herein, because another component extends for a third preset length L3 along the beam axis X and extends for a third preset width D3 along the radial direction of the beam axis X in other embodiments described below. A material of the radiation shield 1315 includes at least one of a photon shielding material and a neutron shielding material. The material may be a rigid solid cut into an appropriate size, such as lead-antimony alloy, Teflon, graphite, paraffin, polyethylene (PE), boron carbide or lithium carbonate or lithium fluoride containing PE, polymethyl methacrylate (PMMA), boron carbide or lithium carbonate or lithium fluoride containing PMMA or boron-containing barite concrete. The material may also be powder filled in a rigid container or a flexible container cut into an appropriate size, such as boron carbide or lithium carbonate or lithium fluoride powder, may also be a liquid filled in a rigid container or a flexible container cut into an appropriate size, such as water dissolved with boron carbide or lithium carbonate or lithium fluoride powder, heavy water, and boric acid, and may further be a flexible solid, such as rubber or silica gel. In the embodiment, the radiation shield 1315 is preferably made of a material different from the material of the reflector, such as boron-containing barite concrete.

It may be understood that the BSA 131 may further be provided with other components and structures. For example, the BSA includes a thermal neutron absorber configured to absorb thermal neutrons, the beam inlet is not accommodated in the moderator, and the like, provided that a desired epithermal neutron beam can be obtained for the treatment.

The collimator 132 is provided behind the beam outlet 1314 or in the BSA 131. An epithermal neutron beam from the collimator 132 is irradiated onto the irradiated body 200. It may be understood that the collimator 132 may also be removed or replaced by other structures. The neutron beam from the beam outlet 1314 is directly irradiated onto the irradiated body 200.

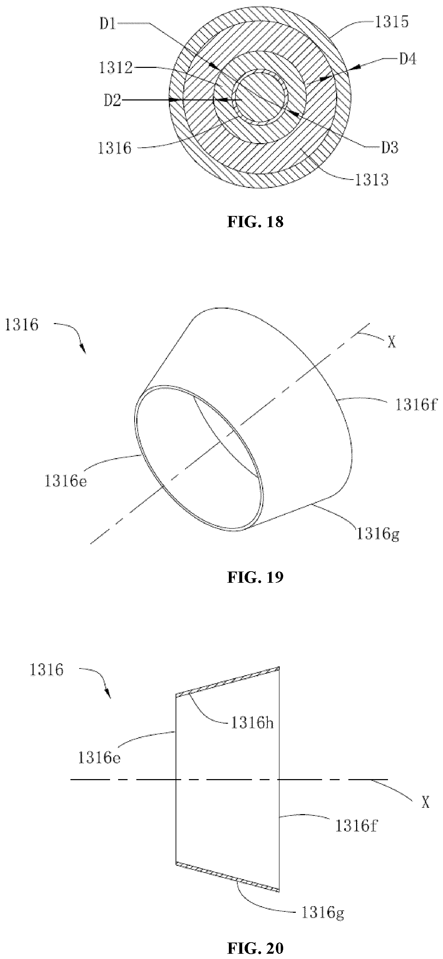

Referring to FIG. 4 to FIG. 21, in order to further improve the flux and quality of the neutron source, the BSA 131 further includes an epithermal neutron flux enhancer 1316. The epithermal neutron flux enhancer 1316 is provided in the moderator 1312 and/or in the reflector 1313 and/or between the moderator 1312 and the reflector 1313. For ease of comparison and description, same components in the embodiment of the present disclosure use a same reference sign.

Referring to FIG. 4 to FIG. 6, the epithermal neutron flux enhancer 1316 extends for a third preset length L3 along the beam axis X and extends for a third preset width D3 along the radial direction of the beam axis X. The third preset width D3 is less than a sum of the first preset width D1 and the second preset width D2. Preferably, in the embodiment shown in FIG. 4 to FIG. 6, the third preset width D3 is less than the first preset width D1. In an embodiment of the present disclosure, the third preset width D3 extended by the epithermal neutron flux enhancer 1316 along the radial direction of the beam axis X includes a thickness of the epithermal neutron flux enhancer. The epithermal neutron flux enhancer 1316 is made of a material with a strong neutron reflectivity. Preferably, the epithermal neutron flux enhancer 1316 is made of Ni, including natural nickel or extracted nickel.

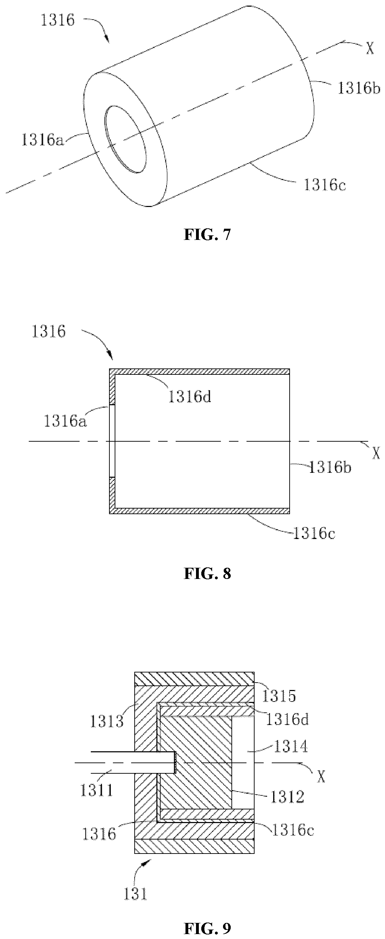

Referring to FIG. 7 to FIG. 8, in combination with FIG. 4 to FIG. 6, the epithermal neutron flux enhancer 1316 is a cylindrical structure. The cylindrical structure includes a first side 1316a and a second side 1316b that are perpendicular to the beam axis X, as well as a first wall 1316c and a second wall 1316d that are enclosed circumferentially around the beam axis X. The first side 1316a and the second side 1316b are arranged in sequence along a direction of the neutron beam N. The first side 1316a is farther away from the beam outlet 1314 than the second side 1316b. In the embodiment, the first side 1316a is provided between the moderator 1312 and the reflector 1313 along the direction of the beam axis X, and adjacent to an end of the moderator 1312 located at an upstream side of the neutron beam N. The first side 1316a is a circular structure, and is provided with a first central hole. The first central hole is configured to allow the beam inlet 1311 to penetrate through, such that the beam inlet 1311 may be accommodated in the moderator 1312. The second side 1316b is provided with a second central hole, adjacent to an end of the BSA 131 located at a downstream side of the neutron beam N, and configured to cooperate with the beam outlet 1314. When a radial size of the second central hole is the same as an outer diameter of the first wall 1316c, the second side 1316b is open without an enclosed structure. The first wall 1316c and the second wall 1316d are arranged along the radial direction of the beam axis X, and perpendicular to the first side 1316a and the second side 1316b. The first wall 1316c and the second wall 1316d are connected to the first side 1316a, and extend to the second side 1316b along the beam axis X. In the embodiment, a central axis of the epithermal neutron flux enhancer 1316 of the cylindrical structure coincides with the axis of the neutron beam N. The third preset width D3 is less than the first preset width D1. At least a portion of the first wall 1316c and at least a portion of the second wall 1316d are accommodated in the moderator 1312. That is, at least a portion of the epithermal neutron flux enhancer 1316 of the cylindrical structure is accommodated in the moderator 1312. It may be understood that the upstream side of the neutron beam N is a side close to the beam inlet 1311 and the target 133, and the downstream side of the neutron beam N is a side close to the beam outlet 1314.

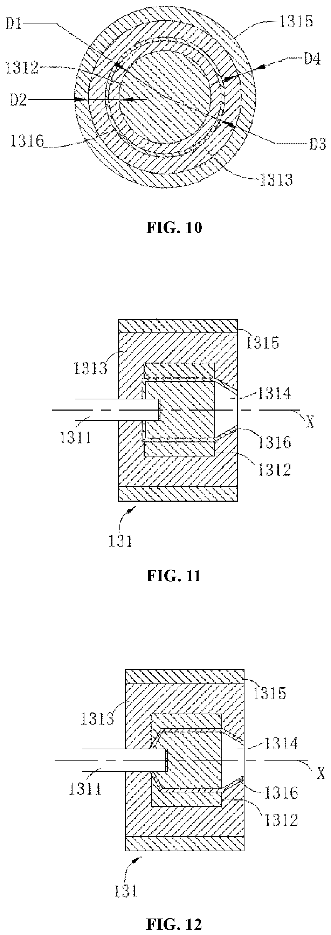

Referring to FIG. 9 and FIG. 10, FIG. 9 and FIG. 10 each are a schematic structural view of a BSA from a different viewing angle according to an embodiment of the present disclosure, where an epithermal neutron flux enhancer 1316 is a cylindrical structure. Different from the embodiment shown in FIG. 4 to FIG. 8, at least a portion of the first wall and at least a portion of the second wall in the epithermal neutron flux enhancer 1316 are provided in the reflector 1313. In the embodiment, the third preset width D3 extended by the epithermal neutron flux enhancer 1316 of the cylindrical structure along the radial direction of the beam axis X is greater than the first preset width D1 extended by the moderator 1312 along the radial direction of the beam axis X, and less than a sum of the first preset width D1 extended by the moderator 1312 along the radial direction of the beam axis X and the second preset width D2 extended by the portion of the reflector 1313 outside the moderator 1312 along the radial direction of the beam axis X. Both the first wall 1316c and the second wall 1316d are adjacent to the reflector 1313. That is, the epithermal neutron flux enhancer 1316 of the cylindrical structure is provided in the reflector 1313.

The following description is made on the beam quality of the embodiments shown by FIG. 3, FIG. 4 to FIG. 8, and FIG. 9 to FIG. 10 in air. Table 1 shows beam quality factors in air when the epithermal neutron flux enhancer is not provided and the epithermal neutron flux enhancer of the cylindrical structure is provided at different positions (the unit of each noun in the table is as described above, is not repeated herein, and is the same below):

Table 1: Beam quality when the epithermal neutron flux enhancer of the cylindrical structure is provided at different positions

| Position of the epithermal neutron flux enhancer | The epithermal neutron flux enhancer is not provided | At least a portion of the epithermal neutron flux enhancer of the cylindrical structure is provided in the moderator | The epithermal neutron flux enhancer of the cylindrical structure is provided in the reflector |
|---|---|---|---|
| Epithermal neutron flux | 9.32 E+08 | 9.40 E+08 | 9.32 E+08 |
| Epithermal neutron current to flux ratio | 0.700 | 0.701 | 0.700 |
| Fast neutron contamination | 1.83 E-13 | 1.80 E-13 | 1.82 E-13 |
| Photon contamination | 7.69 E-13 | 1.03 E-12 | 7.82 E-13 |
| Thermal to epithermal neutron flux ratio | 0.070 | 0.053 | 0.067 |

As can be seen from the above table, by providing the epithermal neutron flux enhancer of the cylindrical structure in the BSA, the quality of the neutron beam can be improved. When the epithermal neutron flux enhancer is provided in the moderator, the epithermal neutron flux is increased more significantly.

Referring to FIG. 11 to FIG. 14, in some embodiments of the present disclosure, the first side 1316a and/or the second side 1316b of the epithermal neutron flux enhancer 1316 of the cylindrical structure is a conical structure narrowed toward the beam axis X. A radial size of an outer contour of the first side 1316a increases gradually along the direction of the neutron beam N. A radial size of an outer contour of the second side 1316b decreases gradually along the direction of the neutron beam N. The radial size of the outer contour of the first side 1316a is at least greater than a radial size of the central hole. The radial size of the outer contour of the second side 1316b is capable of accommodating at least the beam outlet 1314.

Referring also to FIG. 11, FIG. 11 is a schematic structural view of a BSA according to an embodiment of the present disclosure, where one side of an epithermal neutron flux enhancer 1316 of a cylindrical structure is a conical structure. It is to be noted that for better simplicity, some reference signs same as those in the above embodiment are not described in FIG. 11. For example, the first preset width D1, the third preset width D3, the first side 1316a, the second side 1316b, the first wall 1316c, the second wall 1316d and the like may refer to the above embodiment specifically. In other embodiments below, the reference signs are not given in the corresponding figures, and can refer to the description herein. In an embodiment of the present disclosure, the third preset width D3 extended by the epithermal neutron flux enhancer 1316 of the cylindrical structure along the radial direction of the beam axis X is less than the first preset width D1 extended by the moderator 1312 along the radial direction of the beam axis X. At least a portion of the first wall 1316c and at least a portion of the second wall 1316d are accommodated in the moderator 1312. That is, at least a portion of the epithermal neutron flux enhancer 1316 is provided in the moderator 1312. The first side 1316a is adjacent to the end of the moderator 1312 located at the upstream side of the neutron beam N. The second side 1316b is adjacent to the end of the moderator 1312 located at the downstream side of the neutron beam N. The radial size of the outer contour of the second side 1316b decreases gradually along the direction of the neutron beam N from the third preset width D3 to the size of the beam outlet 1314. That is, the second side 1316b is narrowed toward the beam outlet 1314. One side of the epithermal neutron flux enhancer 1316 of the cylindrical structure is the conical structure.

Referring also to FIG. 12, FIG. 12 is a schematic structural view of a BSA according to an embodiment of the present disclosure, where two sides of an epithermal neutron flux enhancer 1316 of a cylindrical structure each are a conical structure. In an embodiment of the present disclosure, the third preset width D3 extended by the epithermal neutron flux enhancer 1316 of the cylindrical structure along the radial direction of the beam axis X is less than the first preset width D1 extended by the moderator 1312 along the radial direction of the beam axis X. At least a portion of the first wall 1316c and at least a portion of the second wall 1316d are accommodated in the moderator 1312. That is, at least a portion of the epithermal neutron flux enhancer 1316 is provided in the moderator 1312. The first side 1316a is adjacent to the end of the moderator 1312 located at the upstream side of the neutron beam N. The radial size of the outer contour of the first side 1316a increases gradually along the direction of the neutron beam N. The radial size of the outer contour of the first side 1316a increases gradually from the radial size of the central hole to the third preset width D3. That is, the first side 1316a is narrowed toward the beam inlet 1311. The second side 1316b is adjacent to the end of the moderator 1312 located at the downstream side of the neutron beam N. The radial size of the outer contour of the second side 1316b decreases gradually along the direction of the neutron beam N. The radial size of the outer contour of the second side 1316b decreases gradually from the third preset width D3 to the size of the beam outlet 1314. That is, the second side 1316b is narrowed toward the beam outlet 1314. Two sides of the epithermal neutron flux enhancer 1316 of the cylindrical structure each are the conical structure.

The following description is made on the beam quality of the embodiments shown by FIG. 3, FIG. 4, FIG. 11 and FIG. 12 in air. Table 2 shows beam quality factors in air when the epithermal neutron flux enhancer is not provided, the epithermal neutron flux enhancer of the cylindrical structure (without the conical structure) is provided, and one side or two sides of the epithermal neutron flux enhancer of the cylindrical structure each are the conical structure:

Table 2: Beam quality when one side or two sides of the epithermal neutron flux enhancer of the cylindrical structure each are the conical structure

| Shape of the epithermal neutron flux enhancer | The epithermal neutron flux enhancer is not provided | The epithermal neutron flux enhancer of the cylindrical structure without the conical structure is provided | One side of the epithermal neutron flux enhancer of the cylindrical structure is the conical structure | Two sides of the epithermal neutron flux enhancer of the cylindrical structure each are the conical structure |
|---|---|---|---|---|
| Epithermal neutron flux | 9.32 E+08 | 9.40 E+08 | 9.67 E+08 | 9.73 E+08 |
| Epithermal neutron current to flux ratio | 0.700 | 0.701 | 0.699 | 0.699 |

(continued)

| Shape of the epithermal neutron flux enhancer | The epithermal neutron flux enhancer is not provided | The epithermal neutron flux enhancer of the cylindrical structure without the conical structure is provided | One side of the epithermal neutron flux enhancer of the cylindrical structure is the conical structure | Two sides of the epithermal neutron flux enhancer of the cylindrical structure each are the conical structure |
|---|---|---|---|---|
| Fast neutron contamination | 1.83 E-13 | 1.80 E-13 | 1.77 E-13 | 1.76 E-13 |
| Photon contamination | 7.69 E-13 | 1.03 E-12 | 1.02 E-12 | 1.01 E-12 |
| Thermal to epithermal neutron flux ratio | 0.070 | 0.053 | 0.052 | 0.051 |

As can be seen from the above table, by providing the epithermal neutron flux enhancer in the BSA and providing one side or two sides of the epithermal neutron flux enhancer of the cylindrical structure as the conical structure, the epithermal neutron flux in the neutron beam can be increased, thereby improving the quality of the neutron beam.

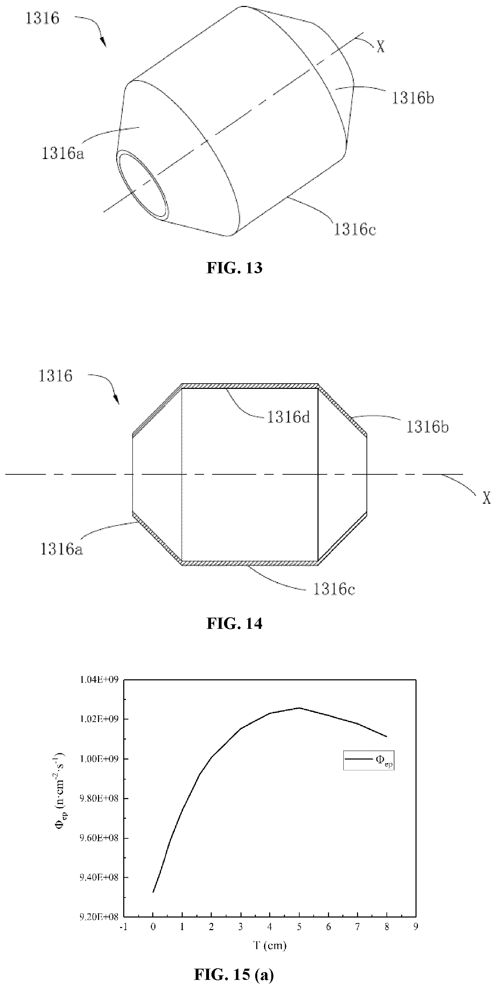

Referring also to FIG. 12, in combination with FIG. 13 and FIG. 14, in an embodiment of the present disclosure, the third preset width D3 extended by the epithermal neutron flux enhancer 1316 of the cylindrical structure along the radial direction of the beam axis X is less than the first preset width D1 extended by the moderator 1312 along the radial direction of the beam axis X. At least a portion of the first wall 1316c and at least a portion of the second wall 1316d are accommodated in the moderator 1312. That is, at least a portion of the epithermal neutron flux enhancer 1316 is provided in the moderator 1312. The first side 1316a is adjacent to the end of the moderator 1312 located at the upstream side of the neutron beam N. The radial size of the outer contour of the first side 1316a increases gradually along the direction of the neutron beam N. The radial size of the outer contour of the first side 1316a increases gradually from the radial size of the central hole to the third preset width D3. That is, the first side 1316a is narrowed toward the beam inlet 1311. The second side 1316b is adjacent to the end of the moderator 1312 located at the downstream side of the neutron beam N. The radial size of the outer contour of the second side 1316b decreases gradually along the direction of the neutron beam N. The radial size of the outer contour of the second side 1316b decreases gradually from the third preset width D3 to the size of the beam outlet 1314. That is, the second side 1316b is narrowed toward the beam outlet 1314. Two sides of the epithermal neutron flux enhancer 1316 of the cylindrical structure each are the conical structure. In the embodiment, a thickness of the epithermal neutron flux enhancer 1316 is less than a sum of the first preset width D1 and the second preset width D2, preferably 1-5 cm, and more preferably 3-5 cm.

The following description is made on the beam quality of the embodiments shown FIG. 3 and FIG. 12 in air when the epithermal neutron flux enhancer 1316 has different thicknesses. Table 3 shows beam quality factors in air when the epithermal neutron flux enhancer is not provided and the epithermal neutron flux enhancer has different thicknesses:

Table 3: Beam quality when the epithermal neutron flux enhancer has different thicknesses

| Thickness of the epithermal neutron flux enhancer (cm) | 0 | 0.2 | 0.4 | 0.6 | 0.8 | 1 | 1.2 | 1.4 |
|---|---|---|---|---|---|---|---|---|
| Epithermal neutron flux | 9.32 E+08 | 9.41E+08 | 9.50E+08 | 9.59E+08 | 9.67E+08 | 9.74E+08 | 9.80E+08 | 9.86E+08 |
| Epithermal neutron current to flux ratio | 0.700 | 6.99E-01 | 6.99E-01 | 6.99E-01 | 6.99E-01 | 6.99E-01 | 6.98E-01 | 6.98E-01 |
| Fast neutron contamination | 1.83 E-13 | 1.82E-13 | 1.80E-13 | 1.79E-13 | 1.77E-13 | 1.76E-13 | 1.75E-13 | 1.74E-13 |
| Photon contamination | 7.69 E-13 | 8.58E-13 | 9.14E-13 | 9.53E-13 | 9.81E-13 | 1.00E-12 | 1.02E-12 | 1.04E-12 |

(continued)

| Thickness of the epithermal neutron flux enhancer (cm) | 0 | 0.2 | 0.4 | 0.6 | 0.8 | 1 | 1.2 | 1.4 |
|---|---|---|---|---|---|---|---|---|
| Thermal to epithermal neutron flux ratio | 7.0E-02 | 6.21E-02 | 5.75E-02 | 5.48E-02 | 5.28E-02 | 5.12E-02 | 5.00E-02 | 4.94E-02 |

Table 3 (continued): Beam quality when the epithermal neutron flux enhancer has different thicknesses

| Thickness of the epithermal neutron flux enhancer (cm) | 1.6 | 1.8 | 2 | 3 | 4 | 5 | 6 | 7 | 8 |
|---|---|---|---|---|---|---|---|---|---|
| Epithermal | 9.92E+0 | 9.97E+0 | 1.00E+0 | 1.02E+0 | 1.02E+0 | 1.03E+0 | 1.02E+0 | 1.02E+0 | 1.01E+0 |
| neutron flux | 8 | 8 | 9 | 9 | 9 | 9 | 9 | 9 | 9 |
| Epithermal neutron current to flux ratio | 6.98E-01 | 6.98E-01 | 6.97E-01 | 6.97E-01 | 6.97E-01 | 6.96E-01 | 6.96E-01 | 6.96E-01 | 6.96E-01 |
| Fast neutron contaminatio n | 1.73E-13 | 1.72E-13 | 1.72E-13 | 1.70E-13 | 1.70E-13 | 1.72E-13 | 1.73E-13 | 1.76E-13 | 1.78E-13 |
| Photon contaminatio n | 1.05E-12 | 1.06E-12 | 1.07E-12 | 1.11E-12 | 1.13E-12 | 1.15E-12 | 1.16E-12 | 1.16E-12 | 1.17E-12 |
| Thermal to epithermal neutron flux ratio | 4.85E-02 | 4.80E-02 | 4.75E-02 | 4.63E-02 | 4.56E-02 | 4.50E-02 | 4.42E-02 | 4.34E-02 | 4.26E-02 |

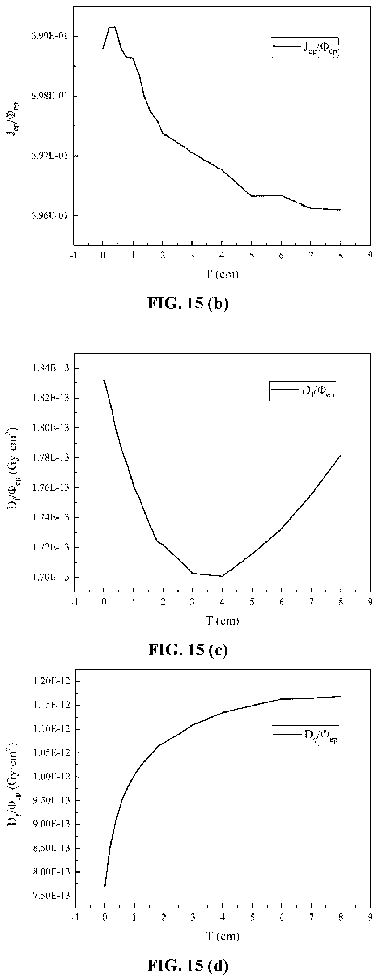

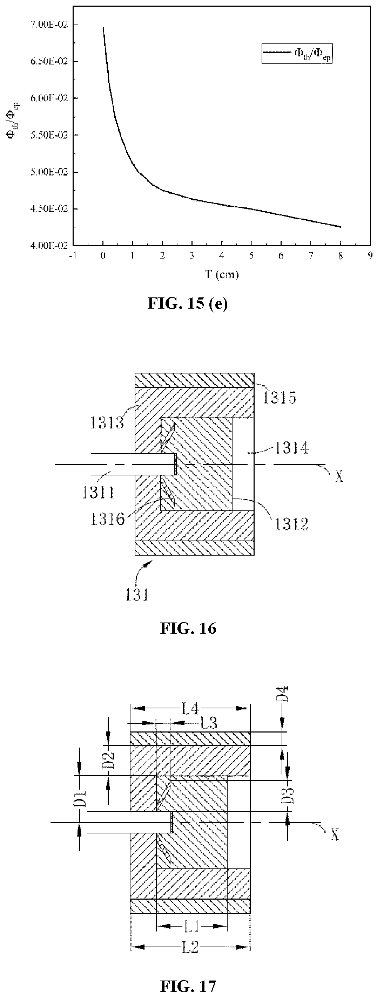

As can be seen from the above table, by providing the epithermal neutron flux enhancer in the BSA and properly increasing the thickness of the epithermal neutron flux enhancer, the epithermal neutron flux in the neutron beam can be increased, thereby improving the quality of the neutron beam. With analysis on FIG. 15 (a) to FIG. 15 (e), when the thickness of the epithermal neutron flux enhancer is 0-5 cm, while the thickness of the epithermal neutron flux enhancer increases, the epithermal neutron flux also increases, and the fast neutron contamination decreases first and then increases. When the thickness of the epithermal neutron flux enhancer is 0-1 cm, the neutron flux increases significantly, and the fast neutron contamination and the thermal to epithermal neutron flux ratio decrease significantly. When the thickness of the epithermal neutron flux enhancer is 1 cm, the beam quality is improved significantly in contrast with the BSA not provided with the epithermal neutron flux enhancer. When the thickness of the epithermal neutron flux enhancer reaches 5 cm, the epithermal neutron flux is maximized. When the thickness of the epithermal neutron flux enhancer is 3 cm and 4 cm, the fast neutron contamination is minimized. If the thickness of the epithermal neutron flux enhancer increases continuously, the epithermal neutron flux decreases, and the fast neutron contamination increases, resulting an unnecessary dose to normal tissues, and affecting the quality of the neutron beam. Hence, the thickness of the epithermal neutron flux enhancer is preferably 1-8 cm, more preferably 3-5 cm. This can obtain the desired epithermal neutron beam with the good beam quality for the neutron capture therapy.

Referring to FIG. 16 to FIG. 22, in some embodiments of the present disclosure, the epithermal neutron flux enhancer 1316 is a conical structure. The conical structure includes a first end 1316e and a second end 1316f that are perpendicular to the beam axis X, as well as a third wall 1316g and a fourth wall 1316h that are enclosed circumferentially around the beam axis X. The first end 1316e and the second end 1316f are arranged in sequence along a direction of the neutron beam N. The third wall 1316g and the fourth wall 1316h are arranged along the radial direction of the beam axis X. The first end 1316e and the second end 1316f each are an opening.

Referring to FIG. 16 to FIG. 20, FIG. 16 to FIG. 20 each are a schematic structural view of a BSA according to an embodiment of the present disclosure, where an epithermal neutron flux enhancer 1316 is a conical structure, and is located at an upstream side of the BSA 131 along a direction of a neutron beam N. In an embodiment of the present disclosure, a radial size of an outer contour of the epithermal neutron flux enhancer 1316 of the conical structure increases gradually along the direction of the neutron beam N. The first end 1316e is adjacent to an end of the moderator 1312 located at an upstream side of the neutron beam N. A size of the first end 1316e is capable of accommodating at least the beam inlet 1311. That is, the epithermal neutron flux enhancer 1316 of the conical structure is provided at the upstream side of the neutron beam N. A radial inner diameter of the first end 1316e is greater than a radial outer diameter of the beam inlet 1311. In the embodiment, the third preset width D3 extended by the epithermal neutron flux enhancer 1316 along the radial direction of the beam axis is less than the first preset width D1 extended by the moderator 1312 along the radial direction of the beam axis. The third wall 1316g and the fourth wall 1316h are accommodated in the moderator 1312. That is, the epithermal neutron flux enhancer 1316 of the conical structure is provided in the moderator 1312, and close to the beam inlet 1311. A central axis of the epithermal neutron flux enhancer 1316 of the conical structure coincides with the beam axis X. The radial size of the outer contour of the epithermal neutron flux enhancer 1316 of the conical structure increases gradually along the direction of the neutron beam N from a size of the beam inlet 1311 to the third preset width D3.

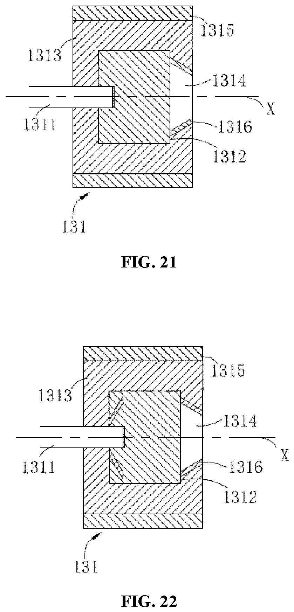

Referring also to FIG. 21, FIG. 21 is a schematic structural view of a BSA according to an embodiment of the present disclosure, where an epithermal neutron flux enhancer 1316 is a conical structure, and is located at a downstream side of the BSA 131 along a direction of a neutron beam N. In an embodiment of the present disclosure, a radial size of an outer contour of the epithermal neutron flux enhancer 1316 of the conical structure decreases gradually along the direction of the neutron beam N. The second end 1316f is adjacent to an end of the BSA 100 located at a downstream side of the neutron beam. A size of the second end 1316f is capable of accommodating at least the beam outlet 1314. That is, the epithermal neutron flux enhancer 1316 of the conical structure is provided at the downstream side of the neutron beam N. A radial inner diameter of the second end 1316f is greater than a radial outer diameter of the beam outlet 1314. In the embodiment, the third preset width D3 extended by the epithermal neutron flux enhancer 1316 along the radial direction of the beam axis X is less than the first preset width D1 extended by the moderator 1312 along the radial direction of the beam axis X. The third wall 1316g is adjacent to the reflector 1313. The fourth wall 1316h is adjacent to the beam outlet 1314. The central line of the epithermal neutron flux enhancer 1316 of the conical structure coincides with the beam axis X. The radial size of the outer contour of the epithermal neutron flux enhancer 1316 of the conical structure decreases gradually along the direction of the neutron beam N from the third preset width D3 to a size of the beam outlet 1314.

Referring also to FIG. 22, FIG. 22 is a schematic structural view of a BSA according to an embodiment of the present disclosure, where an epithermal neutron flux enhancer 1316 is a combination of two conical structures that are respectively located at an upstream side and a downstream side of the BSA 131 along a direction of the neutron beam N. A radial size of an outer contour of the epithermal neutron flux enhancer 1316 of the conical structure at the upstream side increases gradually along the direction of the neutron beam N. The first end 1316e is adjacent to an end of the moderator 1312 located at the upstream side of the neutron beam N. A size of the first end 1316e is capable of accommodating at least the beam inlet 1311. A radial size of an outer contour of the epithermal neutron flux enhancer 1316 of the conical structure at the downstream side decreases gradually along the direction of the neutron beam N. The second end 1316f is adjacent to an end of the BSA 131 located at the downstream side of the neutron beam. A size of the second end 1316f is capable of accommodating at least the beam outlet 1314. In the embodiment, the third preset width D3 extended by the epithermal neutron flux enhancer 1316 along the radial direction of the beam axis X is less than the first preset width D1 extended by the moderator 1312 along the radial direction of the beam axis X. A central line of the epithermal neutron flux enhancer 1316 coincides with the beam axis X. The radial size of the outer contour of the epithermal neutron flux enhancer 1316 of the conical structure at the upstream side increases gradually along the direction of the neutron beam N from a size of the beam inlet 1311 to the third preset width D3. The radial size of the outer contour of the epithermal neutron flux enhancer 1316 of the conical structure at the downstream side decreases gradually along the direction of the neutron beam N from the third preset width D3 to the size of the beam outlet 1314.

The following description is made on the beam quality of the embodiments shown FIG. 3 and FIG. 16 to FIG. 22 in air when the epithermal neutron flux enhancer 1316 has different shapes. Table 4 shows beam quality factors in air when the epithermal neutron flux enhancer is not provided and the epithermal neutron flux enhancer of the conical structure is provided at different positions:

Table 4: Beam quality when the epithermal neutron flux enhancer of the conical structure is provided at different positions

| Position of the epithermal neutron flux enhancer of the conical structure | The epithermal neutron flux enhancer is not provided | The epithermal neutron flux enhancer of the conical structure is provided at the upstream side of the neutron beam | The epithermal neutron flux enhancer of the conical structure is provided at the downstream side of the neutron beam | The epithermal neutron flux enhancer of the conical structure is provided at the upstream side and the downstream side of the neutron beam |
|---|---|---|---|---|
| Epithermal neutron flux | 9.32 E+08 | 9.36 E+08 | 9.92 E+08 | 9.97 E+08 |
| Epithermal neutron current to flux ratio | 0.700 | 0.700 | 0.694 | 0.695 |
| Fast neutron contamination | 1.83 E-13 | 1.83 E-13 | 1.72 E-13 | 1.73 E-13 |
| Photon contamination | 7.69 E-13 | 8.26 E-13 | 7.92 E-12 | 8.40 E-13 |
| Thermal to epithermal neutron flux ratio | 0.070 | 0.065 | 0.063 | 0.058 |

As can be seen from the above table, by providing the epithermal neutron flux enhancer of the conical structure at the downstream end of the BSA along the direction of the neutron beam N, the epithermal neutron flux is increased more significantly, which is more advantageous to improve the quality of the neutron beam.

In the embodiment of the present disclosure, the whole epithermal neutron flux enhancer 1316 is the cylindrical structure or the conical structure or the combination of the cylindrical structure and the conical structure. The whole epithermal neutron flux enhancer 1316 may be formed integrally, and may also be combined by a plurality of components. The epithermal neutron flux enhancer 1316 may be formed integrally with the moderator 1312 and the reflector 1313, such that the epithermal neutron flux enhancer 1316 is provided in the BSA 131. Alternatively, an accommodating cavity may be formed in the moderator 1312 or the reflector 1313, and then the epithermal neutron flux enhancer 1316 is provided in the accommodating cavity, such that the epithermal neutron flux enhancer 1316 is provided in the BSA 131.

The expression "cylindrical" in the embodiment of the present disclosure refers to that the whole outer contour of the structure is basically unchanged from one side to the other side along a direction shown in the figure. One line of the outer contour may be a line segment, such as a corresponding cylindrical line, and may also be an arc close to the line segment with a large curvature, such as a corresponding spherical line with a large curvature. The whole surface of the outer contour may be in smooth transition, and may also be in non-smooth transition, for example, a plurality of bumps and a plurality of grooves are provided on the cylindrical surface or the spherical surface with the large curvature.

The expression "conical" in the embodiment of the present disclosure refers to that the whole outer contour of the structure decreases gradually from one side to the other side along a direction shown in the figure. One line of the outer contour may be a line segment, such as a corresponding conical line, and may also be an arc, such as a corresponding spherical line. The whole surface of the outer contour may be in smooth transition, and may also be in non-smooth transition, for example, a plurality of bumps and a plurality of grooves are provided on the conical surface or the spherical surface.

The technical features of the foregoing embodiments can be employed in arbitrary combinations. For brevity of description, not all possible combinations of the technical features of the foregoing embodiments are described. However, the combinations of the technical features should be construed as falling within the scope described in this specification as long as there is no contradiction in the combinations.

The above described are merely several embodiments of the present invention. Although these embodiments are described specifically and in detail, they should not be construed as a limitation to the patent scope of the present disclosure. It should be noted that those of ordinary skill in the art can further make several variations and improvements without departing from the concept of the present disclosure, and all of these fall within the protection scope of the present disclosure. Therefore, the protection scope of the present disclosure shall be defined by the appended claims.

**Claims**

1. A beam shaping assembly (BSA), configured for a neutron capture therapy system, and comprising:

   a moderator configured to slow a neutron in a neutron beam down to an epithermal neutron, wherein the neutron beam defines a beam axis, and the moderator extends for a first preset length along the beam axis and extends for a first preset width along a radial direction of the beam axis;
   a reflector surrounding the moderator, and configured to reflect a neutron deviated from the neutron beam back to the neutron beam to improve an intensity of the neutron beam, wherein at least a portion of the reflector extends for a second preset length along the beam axis and extends outside the moderator for a second preset width along the radial direction of the beam axis; and
   an epithermal neutron flux enhancer configured to increase an epithermal neutron flux in the neutron beam, and provided in the moderator and/or in the reflector and/or between the moderator and the reflector, wherein the epithermal neutron flux enhancer extends for a third preset length along the beam axis and extends for a third preset width along the radial direction of the beam axis; and the third preset width is less than a sum of the first preset width and the second preset width; and
   further comprising: a beam inlet configured to enter a charged particle beam; and a beam outlet configured to exit the neutron beam, wherein the beam inlet, the moderator, and the beam outlet are arranged along an extension direction of the beam axis.

2. The BSA according to claim 1, further comprising a radiation shield surrounding the reflector, wherein the radiation shield is configured to shield a leaked neutron and a leaked photon to reduce a dose to a normal tissue in a non-irradiated region; and the radiation shield extends for a fourth preset length along the beam axis and extends outside the reflector for a fourth preset width along the radial direction of the beam axis.

3. The BSA according to claim 1, wherein the epithermal neutron flux enhancer is made of Ni.

4. The BSA according to claim 1, wherein the moderator is made of at least one of $D_2O$, $AlF_3$, $CaF_2$, $Li_2CO_3$, $MgF_2$, $Al_2O_3$, and a material mixed by Al, $AlF_3$ and LiF at a preset ratio.

5. The BSA according to claim 1, wherein the reflector is made of lead.

6. The BSA according to claim 1, wherein the epithermal neutron flux enhancer is a cylindrical structure; the epithermal neutron flux enhancer comprises a first side and a second side that are perpendicular to the beam axis, as well as a first wall and a second wall that are enclosed circumferentially around the beam axis; the first side and the second side are respectively provided at two ends of the cylindrical structure in sequence along a direction of the neutron beam; the first side is provided with a first central hole; the first central hole is configured to cooperate with the beam inlet; the second side is provided with a second central hole; and the second central hole is configured to cooperate with the beam outlet.

7. The BSA according to claim 6, wherein the first side and/or the second side of the epithermal neutron flux enhancer is a conical structure narrowed toward the beam axis; a radial size of an outer contour of the first side increases gradually along the direction of the neutron beam; a size of the first central hole is capable of accommodating at least the beam inlet; a radial size of an outer contour of the second side decreases gradually along the direction of the neutron beam; and a size of the second central hole of the second side is capable of accommodating at least the beam outlet.

8. The BSA according to claim 7, wherein the epithermal neutron flux enhancer has a thickness of 1-8 cm.

9. The BSA according to claim 8, wherein the epithermal neutron flux enhancer has a thickness of 3-5 cm.

10. The BSA according to claim 1, wherein the epithermal neutron flux enhancer is a conical structure; the conical structure comprises a first end and a second end that are perpendicular to the beam axis, as well as a third wall and a fourth wall that are enclosed circumferentially around the beam axis; the first end and the second end are arranged in sequence along a direction of the neutron beam; and the first end and the second end each are an opening.

11. The BSA according to claim 10, wherein a radial size of an outer contour of the epithermal neutron flux enhancer increases gradually along the direction of the neutron beam; the first end is adjacent to an end of the moderator located at an upstream side of the neutron beam; and a size of the first end is capable of accommodating at least the beam inlet.

12. The BSA according to claim 10, wherein a radial size of an outer contour of the epithermal neutron flux enhancer decreases gradually along the direction of the neutron beam; the second end is adjacent to an end of the BSA located at a downstream side of the neutron beam; and a size of the second end is capable of accommodating at least the beam outlet.

13. The BSA according to claim 6 or 10, wherein a central line of the cylindrical structure or the conical structure coincides with the beam axis.

14. A neutron capture therapy system, comprising:

a charged particle beam generation portion configured to generate a charged particle beam;
a neutron beam generation portion configured to generate a neutron beam, and comprising a target and a beam shaping assembly (BSA); and
a beam transmission portion configured to transmit the charged particle beam to the neutron beam generation portion, wherein the charged particle beam is interacted with the target to generate the neutron beam; and the neutron beam is moderated by the BSA to form a desired epithermal neutron beam for neutron capture therapy.

15. The neutron capture therapy system according to claim 14, wherein the BSA comprises:

a moderator configured to slow a neutron in the neutron beam down to an epithermal neutron, wherein the neutron beam defines a beam axis, and the moderator extends for a first preset length along the beam axis and extends for a first preset width along a radial direction of the beam axis;
a reflector surrounding the moderator, and configured to reflect a neutron deviated from the neutron beam back to the neutron beam to improve an intensity of the neutron beam, wherein at least a portion of the reflector extends for a second preset length along the beam axis and extends outside the moderator for a second preset width along the radial direction of the beam axis; and
an epithermal neutron flux enhancer configured to increase an epithermal neutron flux in the neutron beam, and provided in the moderator and/or in the reflector and/or between the moderator and the reflector, wherein the epithermal neutron flux enhancer extends for a third preset length along the beam axis and extends for a third preset width along the radial direction of the beam axis; and the third preset width is less than a sum of the first preset width and the second preset width.

**FIG. 1**

$$^1n + {}^{10}B \longrightarrow {}^{11}B^* \longrightarrow {}^7Li + {}^4He + 2.79 \text{ MeV} \qquad (6.1\%)$$

$$\longrightarrow {}^7Li^* + {}^4He + 2.31 \text{ MeV} \qquad (93.9\%)$$

$$\downarrow$$

$$^7Li + \gamma\text{-ray (0.48 MeV)}$$

**FIG. 2**

**FIG. 3**

**FIG. 4**

**FIG. 5**

**FIG. 6**

1316

1316a

1316b

1316c

X

**FIG. 7**

1316

1316a

1316d

1316b

X

1316c

**FIG. 8**

1313

1315

1316d

1314

1311

1312

1316

1316c

131

X

**FIG. 9**

FIG. 10

FIG. 11

FIG. 12

**FIG. 13**

**FIG. 14**

**FIG. 15 (a)**

**FIG. 15 (b)**

**FIG. 15 (c)**

**FIG. 15 (d)**

**FIG. 15 (e)**

**FIG. 16**

**FIG. 17**

**FIG. 18**

**FIG. 19**

**FIG. 20**

**FIG. 21**

**FIG. 22**

## INTERNATIONAL SEARCH REPORT

| | |
|---|---|
| International application No. |
| **PCT/CN2023/122407** |

**A. CLASSIFICATION OF SUBJECT MATTER**

A61N5/10(2006.01)i;  G21K1/00(2006.01)i

According to International Patent Classification (IPC) or to both national classification and IPC

**B. FIELDS SEARCHED**

Minimum documentation searched (classification system followed by classification symbols)

IPC：A61N5/-；G21K1/-

Documentation searched other than minimum documentation to the extent that such documents are included in the fields searched

Electronic data base consulted during the international search (name of data base and, where practicable, search terms used)

DWPI, WPABS, CNTXT, ENTXT, CNKI: 中硼, 中子, 捕获, 射束, 整形, 缓速, 减速, 慢化, 反射, 超热, 通量, 增强, 提高, 镍, neutron, captur+, therapy, beam, shaper, reflect+, retard+, epithermal, flux, Ni

**C. DOCUMENTS CONSIDERED TO BE RELEVANT**

| Category* | Citation of document, with indication, where appropriate, of the relevant passages | Relevant to claim No. |
|---|---|---|
| X | JP 2018161449 A (YAGAMI CO., LTD. et al.) 18 October 2018 (2018-10-18) description, paragraphs [0017]-[0104], and figures 1-6 | 1-15 |
| X | JP 2008022920 A (HITACHI LTD.) 07 February 2008 (2008-02-07) description, paragraphs [0009]-[0038], and figures 1-11 | 1-15 |
| X | CN 114904154 A (NEUBORON THERAPY SYSTEM LTD.) 16 August 2022 (2022-08-16) description, paragraphs [0036]-[0040], and figure 1 | 14 |
| A | JP 2007242422 A (MITSUBISHI HEAVY INDUSTRIES, LTD. et al.) 20 September 2007 (2007-09-20) entire document | 1-15 |
| A | CN 106782738 A (HEFEI INSTITUTES OF PHYSICAL SCIENCE, CHINESE ACADEMY OF SCIENCES) 31 May 2017 (2017-05-31) entire document | 1-15 |
| A | CN 107799195 A (BEIJING XINHE MEDICAL TECHNOLOGY CO., LTD.) 13 March 2018 (2018-03-13) entire document | 1-15 |

☑ Further documents are listed in the continuation of Box C.    ☑ See patent family annex.

| | |
|---|---|
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |
| Date of the actual completion of the international search <br><br> **30 October 2023** | Date of mailing of the international search report <br><br> **08 November 2023** |
| Name and mailing address of the ISA/CN <br><br> **China National Intellectual Property Administration (ISA/CN)** <br> **China No. 6, Xitucheng Road, Jimenqiao, Haidian District, Beijing 100088** | Authorized officer <br><br><br><br><br> Telephone No. |

Form PCT/ISA/210 (second sheet) (July 2022)

<table>
<tr><td colspan="2" align="center">**INTERNATIONAL SEARCH REPORT**</td><td>International application No.<br>**PCT/CN2023/122407**</td></tr>
</table>

**C.     DOCUMENTS CONSIDERED TO BE RELEVANT**

| Category* | Citation of document, with indication, where appropriate, of the relevant passages | Relevant to claim No. |
|---|---|---|
| A | CN 106552323 A (NEUBORON MEDTECH LTD.) 05 April 2017 (2017-04-05)<br>entire document | 1-15 |

Form PCT/ISA/210 (second sheet) (July 2022)

| INTERNATIONAL SEARCH REPORT<br>Information on patent family members | | | | International application No.<br><br>**PCT/CN2023/122407** | | | |
|---|---|---|---|---|---|---|---|

| Patent document<br>cited in search report | | | Publication date<br>(day/month/year) | Patent family member(s) | | | Publication date<br>(day/month/year) |
|---|---|---|---|---|---|---|---|
| JP | 2018161449 | A | 18 October 2018 | JP | 6935878 | B2 | 15 September 2021 |
| JP | 2008022920 | A | 07 February 2008 | JP | 5054335 | B2 | 24 October 2012 |
| CN | 114904154 | A | 16 August 2022 | WO | 2022170986 | A1 | 18 August 2022 |
| | | | | TW | 202245869 | A | 01 December 2022 |
| | | | | KR | 20230106647 | A | 13 July 2023 |
| | | | | CN | 114904155 | A | 16 August 2022 |
| | | | | CN | 114904156 | A | 16 August 2022 |
| | | | | CN | 114904157 | A | 16 August 2022 |
| | | | | CN | 114904158 | A | 16 August 2022 |
| | | | | CN | 114904159 | A | 16 August 2022 |
| | | | | CN | 114904160 | A | 16 August 2022 |
| | | | | CN | 114904161 | A | 16 August 2022 |
| | | | | CN | 114904162 | A | 16 August 2022 |
| | | | | CN | 217448726 | U | 20 September 2022 |
| | | | | CN | 217448727 | U | 20 September 2022 |
| | | | | CN | 217448728 | U | 20 September 2022 |
| | | | | CN | 217745380 | U | 08 November 2022 |
| | | | | CN | 217745381 | U | 08 November 2022 |
| | | | | CN | 218010660 | U | 13 December 2022 |
| | | | | CN | 218010661 | U | 13 December 2022 |
| | | | | CN | 218010662 | U | 13 December 2022 |
| | | | | CN | 218305850 | U | 17 January 2023 |
| JP | 2007242422 | A | 20 September 2007 | JP | 4596392 | B2 | 08 December 2010 |
| CN | 106782738 | A | 31 May 2017 | CN | 106782738 | B | 02 November 2018 |
| CN | 107799195 | A | 13 March 2018 | None | | | |
| CN | 106552323 | A | 05 April 2017 | CN | 205073543 | U | 09 March 2016 |
| | | | | CN | 106552323 | B | 15 May 2018 |
| | | | | CN | 108325095 | A | 27 July 2018 |
| | | | | CN | 108355257 | A | 03 August 2018 |
| | | | | CN | 108543233 | A | 18 September 2018 |
| | | | | CN | 108325095 | B | 07 August 2020 |
| | | | | CN | 108355257 | B | 07 August 2020 |
| | | | | CN | 108543233 | B | 07 August 2020 |
| | | | | WO | 2017054548 | A1 | 06 April 2017 |
| | | | | EP | 3342458 | A1 | 04 July 2018 |
| | | | | EP | 3342458 | B1 | 05 June 2019 |
| | | | | US | 10898733 | B2 | 26 January 2021 |
| | | | | JP | 6722281 | B2 | 15 July 2020 |
| | | | | JP | 2018535717 | A | 06 December 2018 |

Form PCT/ISA/210 (patent family annex) (July 2022)